# EUROPEAN PATENT APPLICATION

(11) **EP 3 872 495 A1**
(43) Date of publication of application: **01.09.2021**
(21) Application number: 19875663.7
(22) Date of filing: 24.10.2019
(51) Int. Cl.: G01N 33/92

(54) **METHOD FOR PROVIDING INFORMATION ON DIAGNOSIS OR PROGNOSTIC MEASUREMENT OF HISTOLOGICAL SEVERITY OF NONALCOHOLIC FATTY LIVER DISEASE**

(30) Priority: 26.10.2018 KR 20180129190
(71) Applicant: Seoul National University Hospital, Seoul 03080 (KR)
(72) Inventor: KIM, Won, Seoul 06601 (KR)
(74) Representative: Plougmann Vingtoft a/s
(86) International application number: PCT/KR2019/014084
(87) International publication number: WO 2020/085820

(57) **Abstract**

The present invention relates to a method for providing information on a diagnosis or prognostic measurement of the histological severity of nonalcoholic fatty liver disease. Since the risk associated with the severity of nonalcoholic fatty liver disease can be considered to have increased when the content of sphingomyelin is at least 1.3 times higher than in a normal control group, the present invention can be effectively used for the diagnosis or prognostic measurement of severity.

## Description

### Technical Field

The present disclosure was made with the support of the Ministry of Health and Welfare of the Republic of Korea, under Project No. 1465025699, which was conducted in the research project named "Analysis of Microbiomes and Serum Metabolites in Faces Through Hepatic histopathological findings in Large-Scale Korean Non-Alcoholic Fatty Liver Prospective Cohort" in the research program entitled "Disease Overcoming Technology Development", by Seoul Metropolitan Government Seoul National University Boramae Medical Center, under the management of the Korean Health Industry Development Institute, 01 January 2018 to 31 December 2018.

This application claims priority to and the benefit of Korean Patent Application No. 10-2018-0129190 filed in the Korean Intellectual Property Office on 26 October 2018, the disclosure of which is incorporated herein by reference.

The present disclosure relates to a method for providing information on diagnosis or prognosis determination of the histological severity of nonalcoholic fatty liver disease and, more specifically, to a method for identifying the increase or decrease of the risk associated with the severity of nonalcoholic fatty liver disease by measuring the levels of saturated sphingomyelins.

### Background Art

Nonalcoholic fatty liver disease (NAFLD) is caused by the abnormal accumulation of hepatic triglycerides (TGs), which is referred to as liver steatosis, and may occasionally progress to nonalcoholic steatohepatitis (NASH) and related fibrosis. Although the excessive accumulation of hepatic TGs is known to initiate NAFLD, the specific types and amounts of lipid classes accumulated in steatotic livers and the impact of lipid alterations on NAFLD progression have not been completely characterized. Since most lipids have important biological activities, the lipid profiling approach offers useful information to help the understanding of NAFLD pathogenesis.

Obesity and insulin resistance are major risk factors for metabolic syndromes, such as NAFLD and type 2 diabetes. However, a considerable number of NAFLD patients have a relatively low body mass index (BMI<25 kg/m²) and less insulin resistance. Since NAFLD is closely associated with obesity, previous metabolomics studies have focused on obese Caucasian adult males with a BMI≥30 kg/m².

However, thin NAFLD patients with healthier metabolic profiles still showed increased total mortality compared with NAFLD patients of overweight or obese groups. Therefore, a better understanding of a metabolic difference between non-obese patients and obese patients both with NAFLD is decisively important in the development of precision medicine and customized therapies based on the various phenotypes and progression levels of NAFLD.

There has been a study that suggests blood metabolite profiles of Western non-obese NAFLD patients with BMI<30 kg/m², but the WHO expert consultation (WHO) recommended that the WHO BMI standards for Asian populations need to be adopted differently from the international classifications.

Although there has been found distinct differences in serum metabolites between non-obese (BMI≤25 kg/kg/m²) and obese (BMI≥30 kg/m²) subjects, the differences between nonalcoholic fatty liver (NAFL) and NASH, which have been histologically confirmed in obese and non-obese NAFLD subjects, need to be further examined.

Therefore, blood lipid distribution variations according to the histological severity of NAFLD need to be comprehensively investigated by using sera obtained from biopsy-proven non-obese and obese Asian adult patients.

### Detailed Description of the Invention

### Technical Problem

The present inventors verified that the histological severity can be diagnosed by measuring the levels of sphingomyelins (SMs) in sera of non-obese nonalcoholic fatty liver disease (NAFLD) patients.

Accordingly, an aspect of the present disclosure is to provide a method for providing information on diagnosis or prognosis determination of the histological severity of nonalcoholic fatty liver disease, the method including a measurement step of measuring the level of a sphingomyelin in a biological sample isolated from a subject.

Another aspect of the present disclosure is to provide use of a sphingomyelin measurement value for providing information of diagnosis or prognosis determination of the histological severity of nonalcoholic fatty liver disease.

### Technical Solution

The present disclosure is directed to a method for providing information on diagnosis or prognosis determination of the histological severity of nonalcoholic fatty liver disease (NAFLD), and the method according to the present disclosure indicates that the increase or decrease of the risk associated with the severity of nonalcoholic fatty liver disease (NAFLD) can be identified by measuring the level of a sphingomyelin (SM) in the serum of a patient.

The present inventors derived that the risk associated with the severity of nonalcoholic fatty liver disease increases when the level of a saturated sphingomyelin is at least 1.3-fold compared with a normal serum.

Hereinafter, the present disclosure will be described in more detail.

An aspect of the present disclosure is to provide a method for providing information on diagnosis or prognosis determination of histological severity of nonalcoholic fatty liver disease (NAFLD), the method including a measurement step of measuring the level of a sphingomyelin (SM) in a biological sample isolated from a subject.

The sphingomyelin may be a saturated sphingomyelin.

The method may further include a risk determination step of determining that the risk associated with the severity of nonalcoholic fatty liver disease of the subject increases when the level of the saturated sphingomyelin in the measurement step is at least 1.3-fold compared with a normal control sample.

Herein, the subject was classified as non-obese when the body mass index of the subject was less than 25 and as obese when the body mass index of the subject was 25 or more.

In the present disclosure, the subject may have a body mass index (BMI) of less than 25. The reason is that specific saturated SMs play a key role in not only the occurrence of nonalcoholic fatty acid but also the progression to NASH in a non-obese group with a body mass index of less than 25, but these features cannot be confirmed in an obese group with a body mass index of 25 or more.

In the present disclosure, the subject may be an Asian. The reason is that a study that presents blood metabolite profiles of Western non-obese NAFLD patients has been disclosed, but the WHO recommended that the WHO BMI standards for the Asian population need to be adopted differently from the international classifications.

In the present disclosure, "Asian" refers to any person having origins in any original peoples of China, Mongolia, Taiwan, Singapore, Korea, Japan, Vietnam, Cambodia, Laos, Burma, Thailand, Malaysia, Indonesia, and Philippines.

In the present disclosure, the biological sample may be a serum or plasma.

In an embodiment of the present disclosure, when the level of a saturated sphingomyelin is measured in the serum isolated from a non-obese Asian patient with a body mass index of less than 25 and the measured sphingomyelin level is increased by at least 1.3-fold compared with a normal control sample, it is determined that the risk associated with the severity of nonalcoholic fatty liver disease increases.

### Advantageous Effects

The present disclosure is directed to a method for providing information on diagnosis or prognosis determination of the histological severity of nonalcoholic fatty liver disease, and the risk associated with the severity of nonalcoholic fatty liver disease is considered to increase when the level of a sphingomyelin is at least 1.3-fold compared with a normal control, and thus the sphingomyelin level can be effectively used in the diagnosis or prognosis determination of the severity.

### Brief Description of the Drawings

FIG. 1A is a graph showing fold changes of lipid species in nonalcoholic fatty liver (NAFL)/no-nonalcoholic fatty disease (no-NAFLD) for non-obese patients.
FIG. 1B is a graph showing fold changes of lipid species in nonalcoholic steatohepatitis (NASH)/NAFL for non-obese patients.
FIG. 1C is a graph showing fold changes of lipid species in NAFL/no-NAFLD for obese patients.
FIG. 1D is a graph showing fold changes of lipid species in NASH/NAFL for obese patients.
FIG. 2A provides graphs showing changes in diacylglycerol (DAG) levels according to the acyl chain length and the degree of desaturation.
FIG. 2B provides graphs showing changes in triacylglycerol (TAG) levels according to the acyl chain length and the degree of desaturation.
FIG. 3 provides graphs showing changes in sphingomyelin (SM) levels in NAFL/no-NAFLD and NASH/NAFL for the non-obese and obese groups.
FIG. 4 provides Spearman's correlation heat maps showing the correlation of SM levels with metabolic risk factors and liver histological severity.
FIG. 5A is a graph showing saturated sphingomyelin levels according to grades of steatosis in the non-obese group.
FIG. 5B is a graph showing saturated sphingomyelin levels according to grades of lobular inflammation in the non-obese group.
FIG. 5C is a graph showing saturated sphingomyelin levels according to grades of ballooning in the non-obese group.
FIG. 5D is a graph showing saturated sphingomyelin levels according to grades of fibrosis in the non-obese group.
FIG. 6A provides graphs showing the liver histological predictive ability of steatosis using the saturated sphingomyelin composition.
FIG. 6B provides graphs showing the liver histological predictive ability of lobular inflammation using the saturated sphingomyelin composition.
FIG. 6C provides graphs showing the liver histological predictive ability of ballooning using the saturated sphingomyelin composition.
FIG. 7 provides graphs showing the histological severity diagnostic performance of NAFLD by combinations of SM d36:0, SM d38:0, and SM d40:0 and aspartate transaminase (AST), alanine transaminase (ALT), and gamma-glutamyl transferase (GGT) in the non-obese (blue curve) and obese (red curve) groups.

### Best Mode for Carrying Out the Invention

The present disclosure is directed to a method for providing information on diagnosis or prognosis determination of the histological severity of nonalcoholic fatty liver disease (NAFLD), the method including a measurement step of measuring the level of a sphingomyelin (SM) in a biological sample isolated from a subject.

### Mode for Carrying Out the Invention

Hereinafter, the present disclosure will be described in more detail by the following examples. However, these examples are used only for illustration, and the scope of the present disclosure is not limited by these examples.

### Example 1: Basic characteristics of study participants

As shown in Tables 1 and 2, subjects were classified according to their BMI as non-obese (<25) and obese (≥25) (diabetes mellitus (DM), metabolic syndrome (MS), high sensitivity C-reactive protein (hsCRP), fasting plasma glucose (FPG), total cholesterol (TC), high density lipoprotein cholesterol (HDL-C)).

**TABLE 1**

| Non-obese (BMI<25) | | | | |
|---|---|---|---|---|
| Parameters | No-NAFLD (n = 48) | NAFL (n = 51) | NASH (n= 31) | *P*-value |
| Age (years) | 53.3±11.9 | 58.6±12.2 | 61.0±9.8 | 0.011 |
| Male, n (%) | 18 (37.5) | 27 (52.9) | 10 (32.3) | 0.853 |
| BMI (kg/m²) | 22.6±1.6 | 23.2±1.3 | 23.6±0.9 | 0.034 |
| Waist circumferenc e (cm) | 81.2±5.5 | 83.1±4.6 | 84.6±3.3 | 0.04 |
| VAT (cm²) | 83.8±35.2 | 110.4±34.2 | 120.5±36.7 | <0.001*** |
| DM, N (%) | 9 (18.8) | 25 (49.0) | 16 (51.6) | <0.001*** |
| MS, N (%) | 16 (33.3) | 29 (56.9) | 25 (80.6) | 0.004 |
| Adipo-IR | 5.1±4.3 | 6.4±4.1 | 9.3±6.4 | <0.001* |
| HOMA-IR | 2.4±1.1 | 3.2±2.1 | 4.1±2.2 | <0.001* |
| HOMA-β | 96.2±52.3 | 83.1±42.4 | 119.8±88.0 | 0.169 |
| HbA1c | 5.7±0.6 | 6.6±1.4 | 6.6±1.0 | <0.001*** |
| hsCRP (mg/dL) | 0.18±0.36 | 0.18±0.35 | 0.30±0.45 | <0.001*^{†} |
| FPG (mg/dL) | 103.2±20.3 | 119.3±35.9 | 117.3±33.9 | 0.009* |
| FFA (mg/dL) | 552.7±279. 8 | 608.7±228. 6 | 674.7±301 | 0.074 |
| TC (mg/dL) | 184.4±37.3 | 182.9±43.4 | 175.7±40.3 | 0.793 |
| TG (mg/dL) | 117±64.6 | 153.1±64.4 | 167.3±74.9 | <0.001** |
| HDL-C (mg/dL) | 52.3±12.8 | 46.1±14.9 | 42.4±10.4 | 0.002* |
| AST (U/L) | 27.5±19.8 | 30.8±17.6 | 55.7±39.4 | <0.001*^{†† †} |
| ALT (U/L) | 24.3±15.3 | 37.6±26.6 | 62.2±49.7 | <0.001** |
| GGT (U/L) | 38.9±40.1 | 44.2±58.8 | 102.8±222. 4 | 0.002^{††} |

**TABLE 2**

| Obese (BMI≥25) | | | | |
|---|---|---|---|---|
| Parameters | No-NAFLD (n = 18) | NAFL (n = 106) | NASH (n= 107) | *P*-value |
| Age (years) | 55.8±9.1 | 49.5±14.5 | 51.2±16.1 | 0.26 |
| Male, n (%) | 7 (38.9) | 65 (61.3) | 48 (44.9) | 0.272^{†} |
| BMI (kg/m²) | 27.8±2.1 | 28.1±2.2 | 29.6±3.2 | 0.002^{††} |
| Waist circumferenc e (cm) | 95.5±5.1 | 94.7±6.1 | 98.8±9.5 | 0.014^{†} |
| VAT (cm²) | 129.9±44.5 | 137.4±42.2 | 154.4±57.2 | 0.015^{†} |
| DM, N (%) | 4 (22.2) | 35 (33.0) | 53 (49.5) | 0.004^{†} |
| MS, N (%) | 8 (44.4) | 78 (73.6) | 89 (83.2) | 0.06 |
| Adipo-IR | 6.0±3.3 | 8.2±4.9 | 14.1±11.1 | <0.001^{†††} |
| HOMA-IR | 2.2±0.7 | 3.8±2.3 | 6.4±5.6 | <0.001^{***†† †} |
| HOMA-β | 83.4±48.8 | 133.6±88.6 | 161.9±176. 1 | 0.003^{**} |
| HbA1c | 5.7±0.4 | 6.0±0.8 | 6.6±1.3 | <0.001^{†††} |
| hsCRP (mg/dL) | 0.25±0.44 | 0.27±1.21 | 0.33±0.42 | <0.001^{†††} |
| FPG (mg/dL) | 107.3±23.3 | 107.5±20.5 | 122.8±44.1 | 0.030^{†} |
| FFA (mg/dL) | 685.6±269. 8 | 608±250.9 | 762+694.5 | 0.036^{†} |
| TC (mg/dL) | 189.3±42.9 | 184.6±42.3 | 185.4±40.2 | 0.956 |
| TG (mg/dL) | 100.2±39.4 | 168.4±102. 1 | 169.6±178. 7 | 0.006** |
| HDL-C (mg/dL) | 58.1±15.0 | 45.2±10.9 | 45.4±11.6 | 0.002** |
| AST (U/L) | 26.9±8.3 | 32.1±14.5 | 59.9±31.8 | <0.001^{†††} |
| ALT (U/L) | 25.2±14.3 | 41.3±27.6 | 80.8±55.2 | <0.001*^{†††} |
| GGT (U/L) | 52.2±53.2 | 46.3±43.6 | 76.6±105.3 | <0.001^{†††} |

As can be confirmed in Tables 1 and 2, serum aspartate aminotransferase (AST), alanine aminotransferase (ALT), and gamma-glutamyl transferase (GGT) levels gradationally increased according to the histological severity of NAFLD in both the non-obese and obese groups.

Among the 361 subjects (mean age 53±14 years, male 48.5%), 295 had biopsy-proven NAFLD and 66 were healthy controls (no-NAFLD group). The no-NAFLD group had no clinical, biochemical, radiological, or histological evidence of fatty liver. Among the non-obese subjects, 82 had NAFLD and 48 were controls. In the obese group, 213 subjects had NAFLD and 18 were controls.

Based on BMI and liver histology findings (no-NAFLD, NAFL, and NASH), study subjects were classified into six groups: non-obese/no-NAFLD; non-obese/NAFL; non-obese/NASH; obese/no-NAFLD; obese/NAFL; and obese/NASH. In both the non-obese and obese groups, as the histological severity of NAFLD increased, the BMI, waist circumference, visceral adipose tissue (VAT) area, insulin resistance in adipose tissue (adipo-IR), insulin resistance (HOMA-IR), glycated haemoglobin (HbA1c), fasting plasma glucose, triglycerides (TG), HDL-cholesterol, the prevalence of diabetes mellitus, and the frequency of metabolic syndrome increased (Table 1).

### Example 2: Serum lipid profiles based on UPLC/QTOF-MS

The subjects with biopsy-proven NAFLD and BMI of less than 25 kg/m² ("Boramae NAFLD cohort" (NCT 02206841)) were defined as non-obese NAFLD subjects. Controls were determined through liver biopsy during the evaluation on a living-donor liver transplant and confirmed to have no NAFLD through characterization of a solid liver mass suspected of liver adenoma or focal nodular hyperplasia based on abdominal image examination.

NAFLD was defined as the presence of 5% or more of macrovesicular steatosis identified via histological examination. NASH was diagnosed based on histological criteria of NASH-clinical research network (CRN): an overall pattern of histological hepatic injury consisting of steatosis, lobular inflammation, ballooning, or fibrosis was graded according to the NAFLD activity storing system.

The visceral adipose tissue (VAT) area was measured for quantification of visceral adipose amounts. Systemic insulin resistance (HOMA-IR) and β-cell function (HOMA-β) were evaluated using HOMA-B, and the insulin resistance in adipose tissue (adipo-IR) was also calculated. The metabolic syndrome was defined by the national cholesterol education program adult treatment panel III (NCEP ATP III) criteria.

The sera of 361 study subjects were analyzed for global lipid profiling by UPLC/Q TOF-MS, and 224 lipid metabolites were identified using several authentic standards and online databases, such as the human metabolome database (HMDB, www.hmdb.ca), METLIN (https://metlin.scripps.edu), and LIPID MAPS (www.lipidmaps.org).

High-performance liquid chromatography (HPLC)-mass spectrometry (MS)-grade solvents for ultra-performance liquid chromatography/quadrupole time-of-flight mass spectrometry (UPLC/QTOF-MS) analysis were purchased from Thermo Fisher Scientific (Waltham, MA). Ammonium acetate and lipid standards were purchased from Sigma-Aldrich (St. Louis, MO).

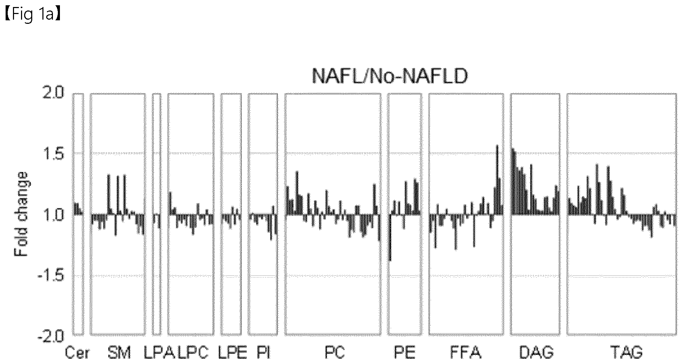

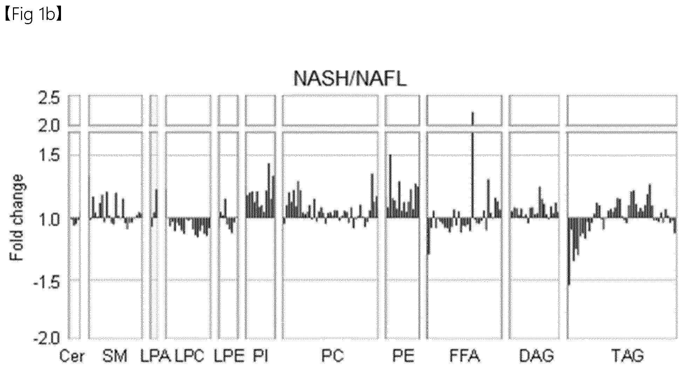

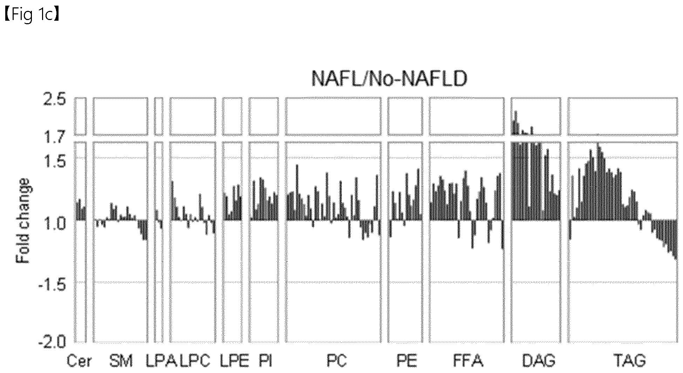

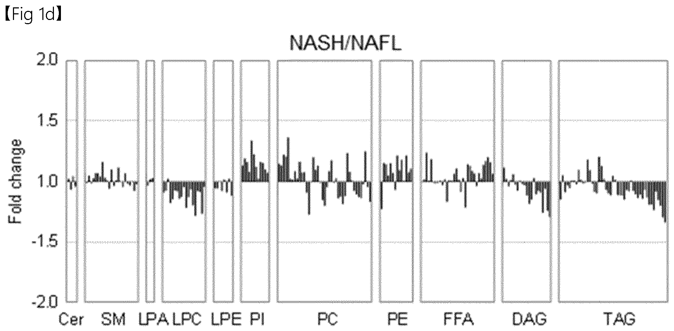

Lipidomic profiles of all lipid classes include: free fatty acids (FFA); glycerolipids (diacylglycerol (DAG) and triacylglycerol (TAG)); glycerophospholipids (lysophosphatidic acid (LPA), lysophosphatidylcholine (LPC), lysophosphatidylethanolamine (LPE), phosphatidylcholine (PC), phosphatidylethanolamine (PE), and phosphatidylinositol (PI); and sphingolipids (ceramide (Cer) and SM). Fold changes of lipid species in NAFL vs no-NAFLD and NASH vs NAFL for non-obese and obese patients are presented in FIG. 1.

As can be confirmed in FIG. 1, several lipid species, such as DAG, TAG, and SM, showed characteristic change patterns according to the histological severity of NAFLD in non-obese and obese adult NAFLD patients.

Especially, in the obese group compared with the non-obese group, the ratios of blood glycerolipid levels (concentrations) in NAFL to normal liver (no-NAFLD) were significantly higher, but the ratios in NASH to NAFL showed no significant differences. As for some specific saturated SMs, the ratios of blood SM levels in NAFL to normal liver (no-NAFLD) and the ratios of blood SM levels in NASH to NAFL were significantly high only in the non-obese group, but showed no significant differences in the obese group.

These data can confirm that in the obese group, the blood glycerolipids play an important role in the occurrence of non-alcoholic fatty liver, but play no great role in the progression to NASH or, rather, triacylglycerol serves as a preventive protective role.

However, in the non-obese group, specific saturated SMs play a significant role in not only the occurrence of nonalcoholic fatty liver but also the progression to NASH, but these features could not be confirmed in the obese group.

### Example 3: DAG and TAG changes depending on NAFLD severity

50 µL of each serum sample was mixed with 500 µL of chloroform/methanol (2:1, v/v). After centrifugation at 13,000 rpm for 20 min at 4°C, 300 µL of the lower lipid phase was collected, and the solvents were removed under a gentle nitrogen stream at room temperature. The dried extracts were reconstituted in 250 µL of an isopropanol/acetonitrile/water mixture (2:1:1, v/v/v). After centrifugation at 13,000 rpm for 10 min at 4°C, the supernatants were transferred to vials for lipidomic analysis using UPLC (ACQUITY™ UPLC system, Waters, Manchester, UK) coupled with quadrupole time-of-flight mass spectrometry (QTOF-MS), and measured using QTOF-MS.

The column oven and auto-sampler temperatures were maintained at 40°C and 4°C, respectively. The samples were eluted and separated using an Acquity UPLC BEH C18 column (2.1 µm x 1.7 mm particles, Waters). The LC mobile phase was composed of 10 mM ammonium acetate in an acetonitrile/water mixture (4:6, v/v) (solvent A) and 10 mM ammonium acetate in an acetonitrile/isopropanol mixture (1:9, v/v) (solvent B). Gradient elution began with 40% B, increased to 65% B after 5 min, and then continuously increased until the composition of B was 99% by 10 min.

The composition was held at 99% B for 2 min. The resultant composition was returned to initial conditions after 17 min and was finally maintained for 3 min until equilibration. The flow rates were set to 350 µL/min. Five microliters of lipid extracts were injected into the UPLC/QTOF-MS system.

All samples were pooled in equal amounts to generate a quality control (QC) sample. Solvent blanks and QC sample injections were carried out between every 12 samples to assess analytical reproducibility. Positive and negative ionization modes were applied to each sample by using a hybrid QTOF instrument and a Triple TOF 5600 (AB Sciex, Concord, Canada) fitted with a DuoSpray ion source. The mass range was set to m/z 50-1500.

The following parameters were used for operation: ion spray voltage of 5500 V, source temperature of 500°C, a nebulizer pressure of 50 psi, a drying gas pressure of 60 psi, curtain gas of 30 psi, declustering potential of 90 V, and information-dependent acquisition (IDA) was used to acquire MS/MS spectra for ions.

To obtain the MS/MS spectra, the collision energy and collision energy spread were adjusted as 40 V and 15 V, respectively. The mass accuracy was maintained with an automated calibrant delivery system (AB Sciex) interfaced with the DuoSpray ion source. Lipid metabolites were identified according to available information (accurate mass, fragment ions, and/or retention time) that matched data from online databases (DBs; HMDB, METLIN, LIPID MAPS) and standard compounds.

Distinctive increases in glycerolipids including DAG and TAG species were observed in both the non-obese and obese groups, with a stepwise increase from NAFL to NASH. The differences in DAG and TAG levels with acyl chain lengths, degrees of desaturation, fold changes, and p-values were visualized using bubble plots.

The bubble plots illustrated the correlations among the chain length, degree of desaturation, fold change, and p-value of DAG and TAG through the comparison of histological subgroups. The y-axis represents the degree of desaturation, and the position of the bubbles relative to the x-axis corresponds to the chain length. The color of the bubbles indicates the fold change, and the size of the bubbles represents p-values that were obtained in the Mann-Whitney U-test with the Bonferroni correction.

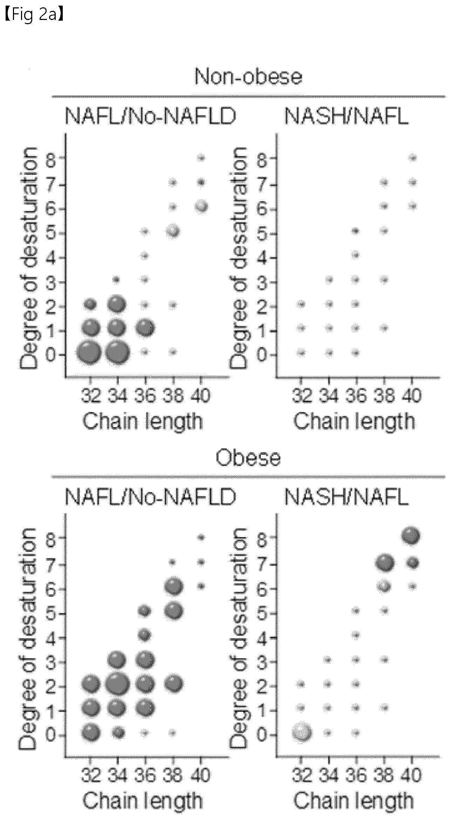

As can be confirmed in FIG. 2A, the changes in DAG levels showed distinctive patterns among various histological subgroups. The levels of DAGs with relatively short chains and low degrees of desaturation statistically increased in NAFL vs NAFLD, regardless of obesity. In contrast, the levels of DAGs with long chains and high degrees of desaturation significantly decreased in NASH vs NAFL for the obese group.

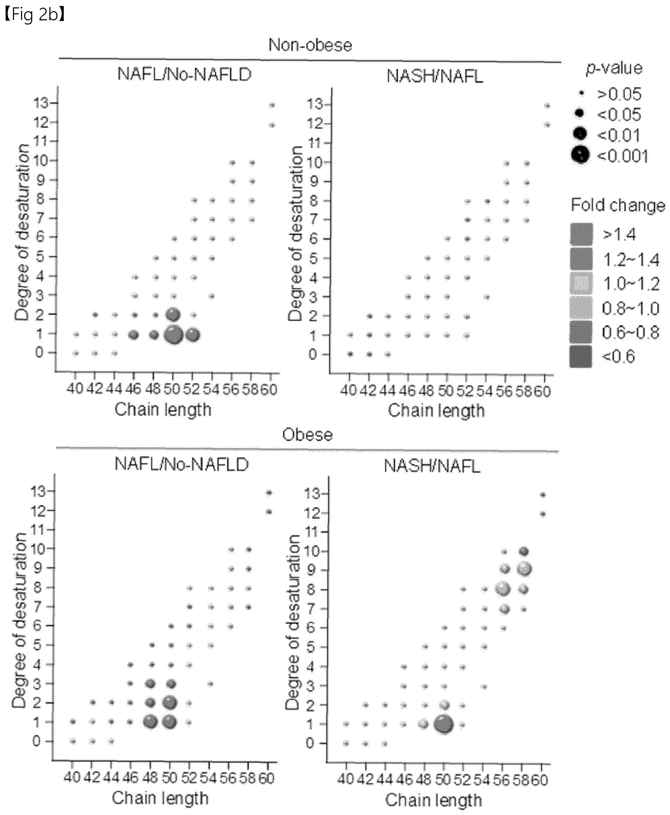

As can be confirmed in FIG. 2B, the changes in TAG levels showed a trend similar to those in DAG levels. The statistical significance and fold change were weakened, but the chain length and the degree of desaturation were slightly changed.

The glycerolipids including DAG and TAG species exhibited distinctive change patterns that were affected by the length and desaturation degree of acyl chains. Moreover, the pattern of glycerolipid changes based on the histological severity was different between the non-obese and obese groups. Resultantly, the fold changes of circulating DAGs and TAGs classified based on histological severity were greater in obese NAFLD subjects than non-obese NAFLD patients.

Especially, the ratios of blood glycerolipid levels (concentrations) in NAFL to normal liver (no-NAFLD) were significantly higher in the obese group compared with the non-obese group, but the ratios of levels in NASH to NAFL showed no great differences.

These results could confirm that in the obese group, the blood glycerolipids play an important role in the occurrence of nonalcoholic fatty liver, but no significant role in the progression to NASH, or, rather triacylglycerol, which is a biologically inactive substance, is a harmless lipid that serves as a preventive protection role.

### Example 4: Changes in metabolic syndromes according to NAFLD severity and correlation of NAFLD severity with liver tissue and liver histology

The differences in intensities of metabolic syndromes according to the histological severity of NAFLD were observed for both the non-obese and obese groups. Significant differences in bar graphs are indicated with asterisks (Mann-Whitney U-test with Bonferroni correction; * p <0.05, ** p <0.01, and *** p <0.001) .

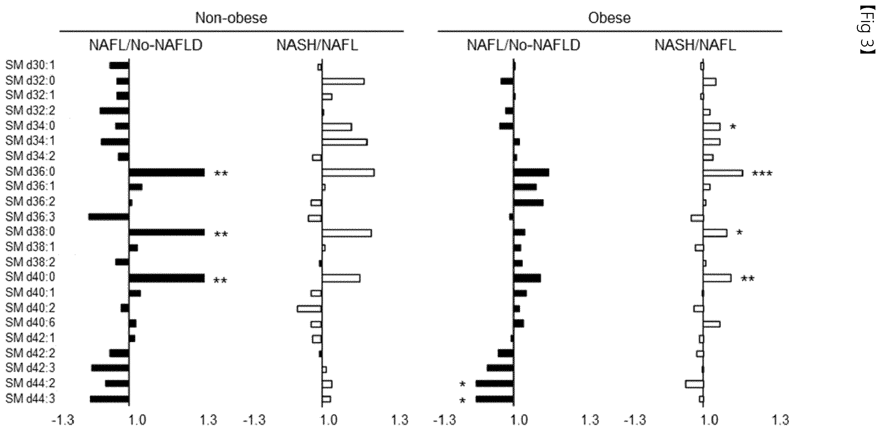

As can be confirmed in FIG. 3, the levels of saturated SMs, including SM d36:0, SM d38:0, and SM d40:0, markedly increased by at least 1.3-fold in the NAFL/no-NAFLD group in the non-obese group. In contrast, the levels of SMs containing a long-chain length of greater than 42 carbons decreased in NAFL/no-NAFLD group, regardless of obesity.

In the obesity group, the levels of saturated SMs, such as SM d34:0, SM d36:0, SM d38:0, and SM d40:0, significantly increased in NASH/NAFL, but were not significant in NAFL/no-NAFLD.

These differences in saturated SM levels associated with NAFLD severity and obesity were also confirmed by the correlations with metabolic risk factors and liver histology. Spearman's correlation heat maps show the correlation of metabolic risk factors or liver histology with the SM levels in the non-obese and obese groups. Statistical significance is indicated with asterisks (* p <0.05, ** p <0.01, and *** p <0.001) (visceral adipose tissue (VAT); glycated haemoglobin (HbA1c); insulin resistance in adipose tissue (adipo-IR): homeostasis model assessment of insulin resistance (HOMA-IR); homeostasis model assessment of β-cell function (HOMA-β); and lobular inflammation (LI)).

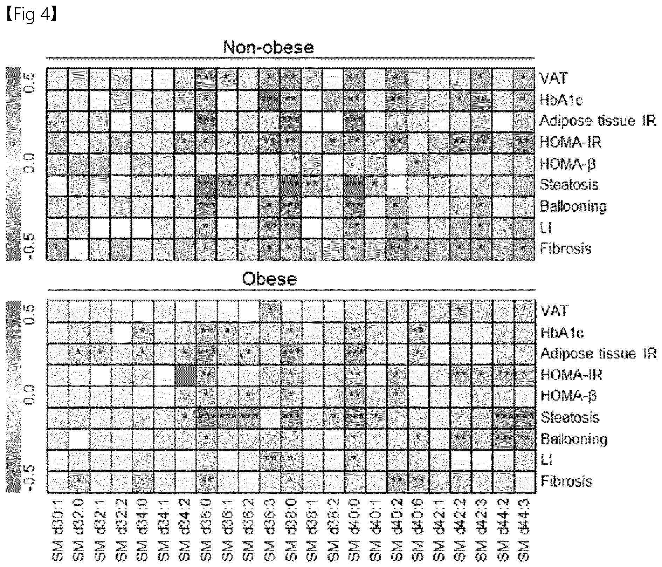

As can be confirmed in FIG. 4, the levels of SM d36:0, SM d38:0, and SM d40:0 were significantly positively correlated with the VAT area, adipo-IR, and HOMA-IR in the non-obese group and were positively correlated with adipo-IR and HOMA-IR in the obese group. Furthermore, saturated SM levels were strongly correlated with the severity of steatosis, ballooning, and lobular inflammation in the non-obese group. However, only the steatosis was positively correlated with saturated lipid levels and amounts in the obese group.

Next, it was examined whether the levels of saturated SM species, including SM d36:0, SM d38:0, and SM d40:0, were altered by the presence and severity of steatosis and lobular inflammation. The intensities of SM d36:0, SM d38:0, and SM d40:0, steatosis (A; 0-3), lobular inflammation (B; 0-3), and ballooning (C; 0-2), but there were no fibrosis stages (D; 0-4) in non-obese subjects. Data are expressed as the means ± standard deviation. Statistical significance is indicated with asterisks (Jonckheere-Terpstra test, * p <0.05, ** p <0.01, and *** p <0.001).

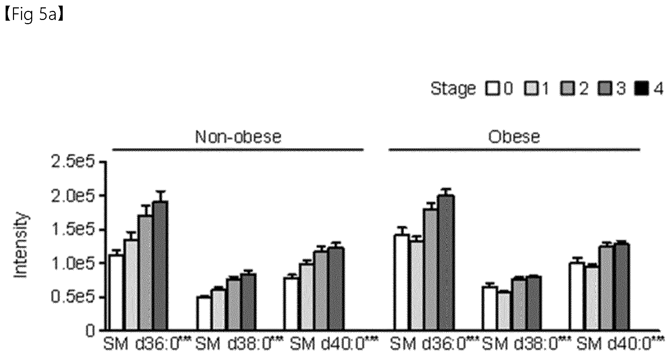

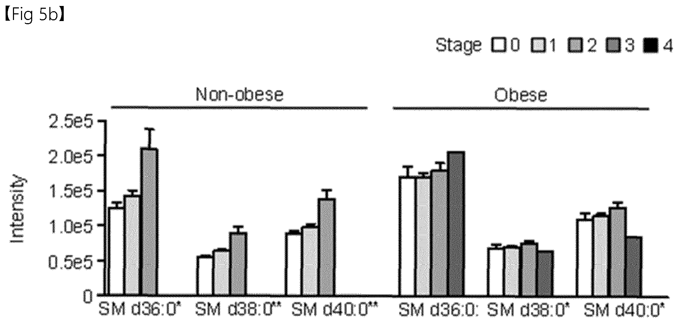

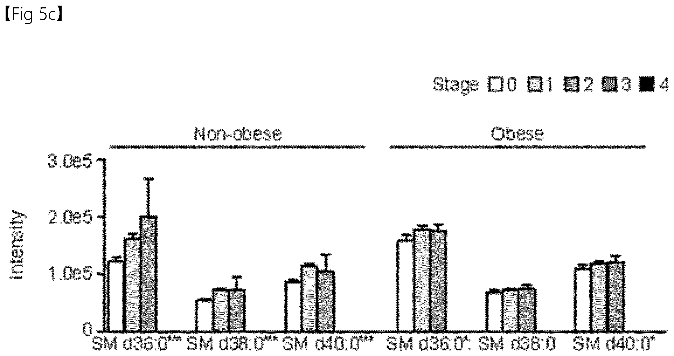

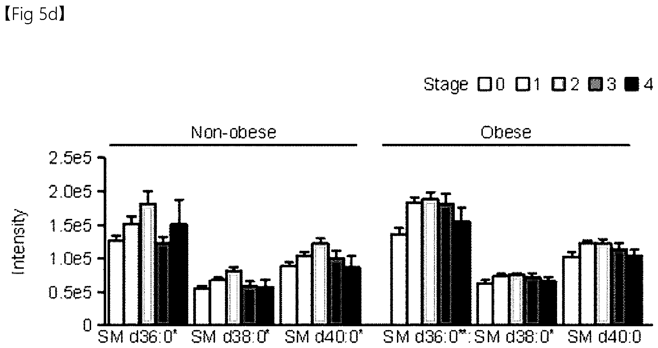

As can be confirmed in FIG. 5, as seen on the heat maps, the levels of saturated SMs markedly increased depending on the severity of steatosis, lobular inflammation, ballooning, and fibrosis according to grades thereof, especially in the non-obese patients. However, the saturated SM levels in the obese patients showed no stepwise increase with respect to the measurement of the histological severity of NAFLD except for the measurement of grades of steatosis. The fibrosis stage also had no stepwise association with the saturated SM levels regardless of obesity.

The saturated SM levels were further useful in improving diagnostic performance of NAFL and NASH in non-obese patients rather than obese NAFL and NASH, thereby preventing liver biopsy especially unnecessary for non-obese adult NAFLD patients.

### Example 5: Predicting NAFLD using circulating saturated SM levels

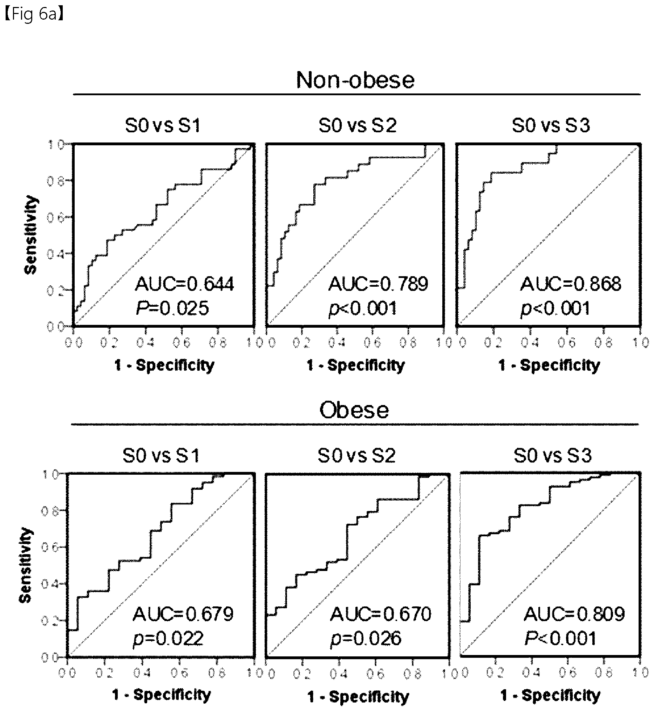

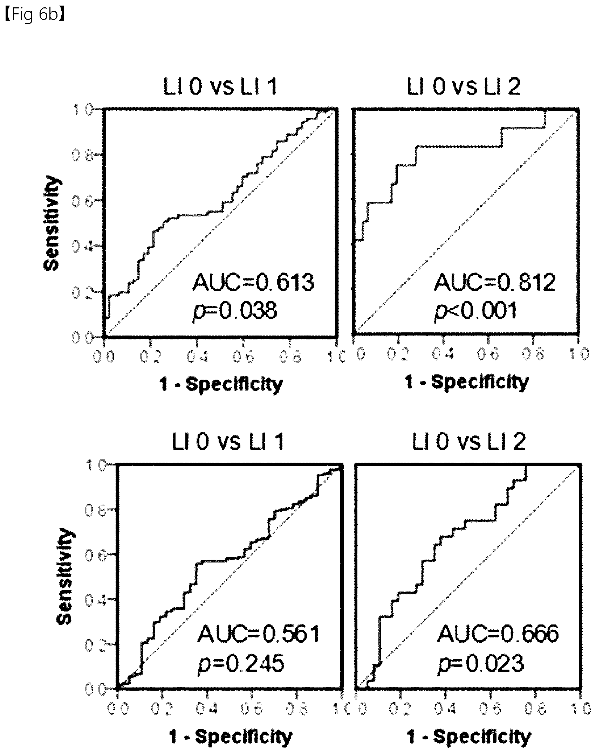

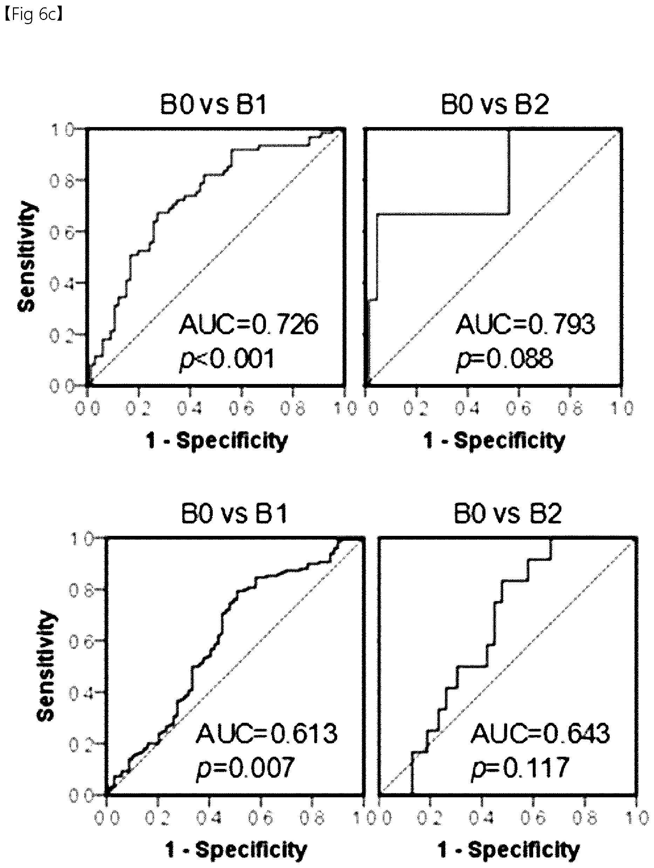

To predict the histological severity of NAFLD by evaluating the diagnostic performance of combinations of the saturated SM levels (SM d36:0, SM d38:0, and SM d40:0), AUROCs with 95% Cl were classified into the non-obese and obese groups and presented in Table 3 and FIG. 6.

**TABLE 3**

| | Area | Error | *p-*value | 95% CI | Sensitivity | Specificity |
|---|---|---|---|---|---|---|
| ROC for | Non-obese | | | | | |
| S1 | 0.644 | 0.062 | 0.025 | 0.522-0.766 | 56% | 56% |
| S2 | 0.789 | 0.057 | <0.001 | 0.677-0.900 | 74% | 73% |
| S3 | 0.868 | 0.046 | <0.001 | 0.778-0.959 | 84% | 81% |
| LI 1 | 0.613 | 0.052 | 0.038 | 0.511-0.715 | 55% | 55% |
| LI 2 | 0.812 | 0.082 | 0.001 | 0.651-0.973 | 75% | 75% |
| B1 | 0.726 | 0.045 | <0.001 | 0.637-0.815 | 69% | 68% |
| B2 | 0.793 | 0.148 | 0.088 | 0.503-1.000 | 67% | 67% |

| ROC for | Obese | | | | | |
|---|---|---|---|---|---|---|
| S1 | 0.679 | 0.072 | 0.022 | 0.538-0.820 | 57% | 56% |
| S2 | 0.67 | 0.067 | 0.026 | 0.538-0.803 | 56% | 56% |
| S3 | 0.809 | 0.057 | <0.001 | 0.697-0.920 | 72% | 72% |
| LI 1 | 0.561 | 0.051 | 0.245 | 0.461-0.662 | 57% | 57% |
| LI 2 | 0.666 | 0.067 | 0.023 | 0.534-0.798 | 64% | 65% |
| B1 | 0.613 | 0.043 | 0.007 | 0.528-0.698 | 59% | 57% |
| B2 | 0.643 | 0.066 | 0.117 | 0.513-0.772 | 58% | 57% |

As can be confirmed in FIG. 6A, in the non-obese group, the AUROCs for steatosis 1, 2, and 3 (S1-S3) were 0.720, 0.768, and 0.804, respectively. In the obese group, the AUROCs for S1, S2, and S3 were 0.722, 0.656, and 0.733, respectively.

As can be confirmed in FIG. 6B, as for lobular inflammation, in the non-obese group, the AUROCs for lobular inflammation were 0.613 (score 1) and 0.821 (score 2), and in the obese group, the AUROCs were 0.541 and 0.614.

As can be confirmed in FIG. 6C, as for ballooning, in the non-obese group, the AUROCs for ballooning were 0.709 (score 1) and 0.784 (score 2). In the obese group, the AUROCs for scores 1 and 2 were 0.567 and 0.551, respectively.

In general, the ROC curves based on the histological severity in the non-obese group showed significantly high AUROCs compared with the obese group.

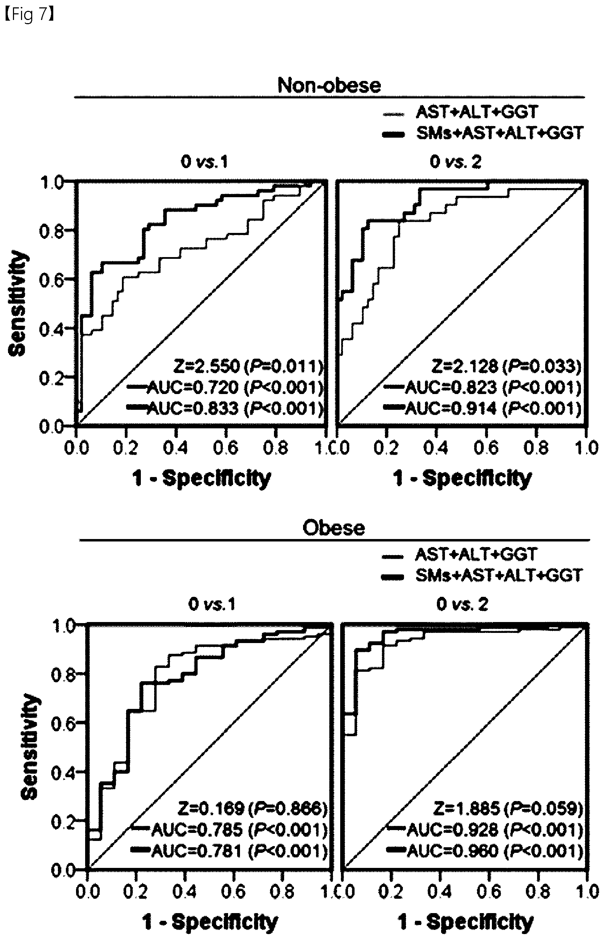

As can be confirmed in FIG. 7, to effectively discriminate subjects with NASH or NAFL from patients without NAFLD, the AUROCs were adjusted using combinations of AST, ALT, and GGT levels (model 1) or combinations of saturated SM levels (SM d36:0, SM d38:0, and SM d40:0) with AST, ALT, and GGT levels (model 2). P-values for pairwise comparison of AUROCs were provided using DeLong test.

**TABLE 4**

| | Area | Error | *p-*value | 95% CI | Sensitivity | Specificity |
|---|---|---|---|---|---|---|
| Combinations of AST, ALT, and GGT | | | | | | |
| ROC for | Non-obese | | | | | |
| 0 v*s.*1 | 0.720 | 0.051 | <0.001 | 0.619-0.821 | 65% | 67% |
| 0 *vs*.2 | 0.823 | 0.049 | <0.001 | 0.726-0.919 | 77% | 77% |
| 1 *vs*.2 | 0.796 | 0.053 | <0.001 | 0.693-0.899 | 77% | 75% |

| ROC for | Obese | | | | | |
|---|---|---|---|---|---|---|
| 0 v*s.*1 | 0.785 | 0.061 | <0.001 | 0.665-0.906 | 73% | 72% |
| 0 *vs.*2 | 0.928 | 0.029 | <0.001 | 0.871-0.986 | 84% | 83% |
| 1 *vs*.2 | 0.836 | 0.027 | <0.001 | 0.783-0.890 | 78% | 76% |

| Combinations of saturated SMs (SM d36:0, SM d38:0, and SM d40:0), AST, ALT, and GGT | | | | | | |
|---|---|---|---|---|---|---|
| ROC for | Non-obese | | | | | |
| 0 v*s.*1 | 0.833 | 0.04 | <0.001 | 0.753-0.914 | 73% | 73% |
| 0 *vs.*2 | 0.914 | 0.031 | <0.001 | 0.852-0.976 | 84% | 83% |
| 1 *vs*.2 | 0.808 | 0.051 | <0.001 | 0.709-0.907 | 68% | 69% |

| ROC for | Obese | | | | | |
|---|---|---|---|---|---|---|
| 0 v*s.*1 | 0.781 | 0.06 | <0.001 | 0.663-0.899 | 76% | 78% |
| 0 *vs*.2 | 0.96 | 0.022 | <0.001 | 0.916-1.000 | 90% | 89% |
| 1 *vs*.2 | 0.837 | 0.027 | <0.001 | 0.784-0.889 | 77% | 75% |

As can be confirmed in Table 4, in the non-obese group, the AUROCs for models 1 and 2 with comparison of NAFLD and NAFL were 0.720 and 0.833 (p=0.011 for AUROCs comparison). When the serum saturated SMs were added to serum AST, ALT, and GCT combinations, the diagnostic performance for distinguishing NASH from NAFL was significantly improved in the non-obese group.

The AUROCs for models 1 and 2 with comparison of NAFLD and NASH were 0.823 and 0.914 (p=0.033 for AUROCs comparison). The AUROCs for models 1 and 2 with comparison of NAFLD and NAFL were 0.785 and 0.781 (p=0.866 for AUROCs comparison). The AUROCs for models 1 and 2 with comparison of NAFLD and NASH were 0.928 and 0.960 (p=0.059 for AUROCs comparison).

### Industrial Applicability

The present disclosure relates to a method for providing information on diagnosis or prognosis determination of the histological severity of nonalcoholic fatty liver disease and, more specifically, to a method for identifying the increase or decrease of the risk associated with the severity of nonalcoholic fatty liver disease by measuring the levels of saturated sphingomyelins.

## Claims

1. A method for providing information on diagnosis or prognosis determination of histological severity of nonalcoholic fatty liver disease (NAFLD), the method comprising a measurement step of measuring the level of a sphingomyelin (SM) in a biological sample isolated from a subject.

2. The method of claim 1, wherein the sphingomyelin is a saturated sphingomyelin.

3. The method of claim 2, further comprising a risk determination step of determining that the risk associated with the severity of nonalcoholic fatty liver disease of the subject increases when the level of the saturated sphingomyelin in the measurement step is at least 1.3-fold compared with a normal control sample.

4. The method of claim 1, wherein the subject has a body mass index (BMI) of less than 25.

5. The method of claim 1, wherein the subject is an Asian.

6. The method of claim 1, wherein the biological sample is a serum or plasma.
